(19) **Europäisches Patentamt**
**European Patent Office**
**Office européen des brevets**

(11) **EP 2 733 634 A1**

(12) **EUROPEAN PATENT APPLICATION**

(43) Date of publication:
**21.05.2014 Bulletin 2014/21**

(51) Int Cl.:
***G06F 19/20*** *(2011.01)* ***G06F 19/24*** *(2011.01)*

(21) Application number: **12192985.5**

(22) Date of filing: **16.11.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Siemens Aktiengesellschaft**
**80333 München (DE)**

(72) Inventors:
- **Keller, Andreas**
  **66346 Püttlingen (DE)**
- **Stähler, Cord Friedrich**
  **69493 Hirschberg an der Bergstraße (DE)**

(54) **Method for obtaining gene signature scores**

(57) The invention relates to a method for evaluating a sample of a patient and to a computer program product useful for performing the method of the invention. The invention provides an easy to use method to obtain a combined score of a plurality of p markers based upon their differential upregulation or downregulation in a sample or patient of interest relative to a control sample or subject.

## FIG 2

**Description**

The invention relates to a method for evaluating a sample of a patient and to a computer program product useful for performing the method of the invention.

Background of the invention

In diagnostics, the expression level of certain markers (i.e. marker genes, nucleic acids, expressed proteins, metabolites, etc.) can be indicative of a physiological or pathological condition and thus be used to arrive at a diagnosis.

Very recently, molecular diagnostics has increasingly gained in importance. It has found an entry into the clinical diagnosis of diseases (inter alia detection of infectious pathogens, detection of mutations of the genome, detection of diseased cells and identification of risk factors for predisposition to a disease).

In particular, through the determination of gene expression in patient samples, nucleic acid analysis opens up very promising new possibilities in the study and diagnosis of disease.

Nucleic acids of interest to be detected include genomic DNA, expressed mRNA and other RNAs such as MicroRNAs (abbreviated miRNAs). MiRNAs are a new class of small RNAs with various biological functions (A. Keller et al., Nat Methods. 2011 8(10):841-3).

In gene expression analysis, often the analysis of the expression level of a single marker is not sufficient to obtain a statistically significant differentiation between e.g. a physiological or pathological condition. It is frequently necessary to analyze a plurality of expression levels of a respective plurality of markers and combine this information to obtain an indication of a physiological or pathological condition. Generally for many tests a trend towards complex patterns instead of single markers can be recognized. Among the most popular examples are the genetic tests of Genomic Health and Agendia, being tests for breast cancer detection from 50-80 genes. A high entry barrier for such tests is the complex biostatistical methodology such as support vector machines. Clinicians are not used to apply such tools while they are used to cope with easily understandable scores.

Therefore, there is a need to provide a simple and effective method for obtaining a combined score form a plurality of markers.

Definitions

Unless defined otherwise, technical and scientific terms used herein have the same meaning as commonly understood by one of ordinary skill in the art to which this invention belongs.

The term "predicting an outcome" of a disease, as used herein, is meant to include both a prediction of an outcome of a patient undergoing a given therapy and a prognosis of a patient who is not treated.

An "outcome" within the meaning of the present invention is a defined condition attained in the course of the disease. This disease outcome may e.g. be a clinical condition such as "relapse of disease", "remission of disease", "response to therapy", a disease stage or grade or the like.

A "risk" is understood to be a probability of a subject or a patient to develop or arrive at a certain disease outcome. The term "risk" in the context of the present invention is not meant to carry any positive or negative connotation with regard to a patient's wellbeing but merely refers to a probability or likelihood of an occurrence or development of a given event or condition.

The term "clinical data" relates to the entirety of available data and information concerning the health status of a patient including, but not limited to, age, sex, weight, menopausal/hormonal status, etiopathology data, anamnesis data, data obtained by in vitro diagnostic methods such as blood or urine tests, data obtained by imaging methods, such as x-ray, computed tomography, MRI, PET, spect, ultrasound, electrophysiological data, genetic analysis, gene expression analysis, biopsy evaluation, intraoperative findings.

The term "evaluating of a sample" of a patient, as used herein, relates to the association of said sample with a value number, such as a score. Said value number may correlate with an outcome of disease, a diagnosis, a risk or the like. As an example, a plurality of p parameters (wherein p is an integer and each individual parameter can be assigned a corresponding individual value) determined from the sample may be combined to yield such a value number.

The term "classification of a sample" of a patient, as used herein, relates to the association of said sample with at least one of at least two categories. These categories may be for example "high risk" and "low risk"; or high, intermediate and low risk; wherein risk is the probability of a certain event occurring in a certain time period, e.g. occurrence of disease, progression of disease, etc. It can further mean a category of favourable or unfavourable clinical outcome of disease, responsiveness or non-responsiveness to a given treatment, diagnosis or the like. Classification may be performed by use of an algorithm, in particular a discriminant function. Classification of a sample of a patient may be used to predict an outcome of disease or the risk of developing a disease. Instead of using the expression level of a single nucleic acid of interest, a combined score of several nucleic acids of interest of interest may be used. Further, additional data may be used in combination. Such additional data may be clinical data from the patient, such as sex, age, weight of the patient, disease grading etc.

A "discriminant function" is a function of a set of variables used to classify an object or event. A discriminant function thus allows classification of a patient, sample or event into a category or a plurality of categories according to data or parameters available from said patient, sample or event. Such classification is a standard instrument of statistical analysis well known to the skilled person. E.g. a patient may be classified as "high risk" or "low risk", "in need of treatment" or "not in need of treatment" or other categories according to data obtained from said patient, sample or event. Classification is not limited to "high vs. low", but may be performed into a plurality of categories, grading or the like.

The term "marker" or "biomarker" refers to a biological molecule, e.g., a nucleic acid, peptide, protein, hormone, etc., whose presence or concentration can be detected and correlated with a known condition, such as a disease state, or with a clinical outcome, such as response to a treatment.

The term "expression level" refers, e.g., to a determined level of expression of a nucleic acid of interest. The level of expression indicates the amount of expression product in a sample or in a given volume. The expression product of a nucleic acid of interest can be the nucleic acid of interest itself, a nucleic acid transcribed or derived therefrom, a polypeptide or protein derived therefrom, or a metabolic product derived therefrom. The term "pattern of expression levels" refers to a determined level of expression compared either to a reference nucleic acid, e.g. from a control, or to a computed average expression value, e.g. in DNA-chip analyses. A pattern is not limited to the comparison of two genes but is also related to multiple comparisons of genes to reference genes or samples. A certain "pattern of expression levels" may also result and be deter-mined by comparison and measurement of several nucleic acids of interest disclosed hereafter and display the relative abundance of these transcripts to each other. Expression levels may also be assessed relative to expression in different tissues, patients versus healthy controls, etc.

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

In the context of the present invention a "sample" or a "biological sample" is a sample which is derived from or has been in contact with a biological organism. Examples for biological samples are: cells, tissue, body fluids, biopsy specimens, blood, urine, saliva, sputum, plasma, serum, cell culture supernatant, and others.

A "control sample" is a sample obtained from a sample source serving as a point of reference. Examples include a non-diseased or healthy subject, non-diseased tissue from a patient suffering from said disease (e.g. non-cancerous tissue vs. cancerous tissue), a standardized calibrator (such bas a reference solution having a defined concentration of an expression product of a nucleic acid of interest) and the like.

A "probe" is a molecule or substance capable of specifically binding or interacting with a specific biological molecule. The term "primer", "primer pair" or "probe", shall have ordinary meaning of these terms which is known to the person skilled in the art of molecular biology. In a preferred embodiment of the invention "primer", "primer pair" and "probes" refer to oligonucleotide or polynucleotide molecules with a sequence identical to, complementary too, homologues of, or homologous to regions of the target molecule or target sequence which is to be detected or quantified, such that the primer, primer pair or probe can specifically bind to the target molecule, e.g. target nucleic acid, RNA, DNA, cDNA, gene, transcript, peptide, polypeptide, or protein to be detected or quantified. As understood herein, a primer may in itself function as a probe. A "probe" as understood herein may also comprise e.g. a combination of primer pair and internal labeled probe, as is common in many commercially available qPCR methods.

A "miRNA" is a short, naturally occurring RNA molecule and shall have the ordinary meaning understood by a person skilled in the art. A "molecule derived from a miRNA" is a molecule which is chemically or enzymatically obtained from an miRNA template, such as cDNA.

The term "array" refers to an arrangement of addressable locations on a device, e.g. a chip device. The number of locations can range from several to at least hundreds or thousands. Each location represents an independent reaction site. Arrays include, but are not limited to nucleic acid arrays, protein arrays and antibody-arrays. A "nucleic acid array" refers to an array containing nucleic acid probes, such as oligonucleotides, polynucleotides or larger portions of genes. The nucleic acid on the array is preferably single stranded. A "microarray" refers to a biochip or biological chip, i.e. an array of regions having a density of discrete regions with immobilized probes of at least about 100/cm2.

A "PCR-based method" refers to methods comprising a polymerase chain reaction PCR. This is a method of exponentially amplifying nucleic acids, e.g. DNA or RNA by enzymatic replication in vitro using one, two or more primers. For RNA amplification, a reverse transcription may be used as a first step. PCR-based methods comprise kinetic or quantitative PCR (qPCR) which is particularly suited for the analysis of expression levels,). When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). The term "PCR based method" comprises both end-point PCR applications as well as kinetic/real time PCR techniques applying special fluorophors or intercalating dyes which emit fluorescent signals as a function of amplified target and allow monitoring and quantification of the target. Quantification methods could be either absolute by external standard curves or relative to a comparative internal standard.

The term "next generation sequencing" or "high throughput sequencing" refers to high-throughput sequencing technologies that parallelize the sequencing process, producing thousands or millions of sequences at once. Examples include Massively Parallel Signature Sequencing (MPSS) Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing.

Object of the invention

The technical problem underlying the present invention is to provide a simple and effective method for obtaining a combined score form a plurality of markers..

Summary of the invention

Before the invention is described in detail, it is to be understood that this invention is not limited to the particular component parts of the process steps of the methods described as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

The invention provides an easy to use method to obtain a combined score of a plurality of p markers based upon their differential upregulation or downregulation in a sample or patient of interest relative to a control sample or subject.

The invention relates to a method of evaluating a sample of a patient, said method comprising the steps of:

a) determining in said sample an expression level of a plurality of markers to obtain a plurality of expression level values,
b) mathematically combining the plurality of expression level values and comparing the plurality of expression level values to a reference pattern of expression levels of a plurality of control samples,

wherein said step of mathematically combining and comparing is performed by calculating a combined score using the formula

$$Z_{(a)} = 1/p\ [\sum[z_{ai}]\ (i \in upregulated)\ +\ \sum(-1)\ [z_{aj}]\ (j \in down\text{-}regulated)]$$

where Za is the combined score,
$z_{a,i}$ is the score of the patient a for each up-regulated marker i,
$z_{a,j}$ is the z-score for each patient a and each down-regulated marker j,
p is the overall number of markers of said plurality of markers,
and wherein

$$Z_{a,i} = (X_{ai} - \mu_i)/\delta_i$$

and

$$Z_{a,j} = (X_{aj} - \mu_j)/\delta_j$$

where x is the actual expression value of patient a for marker i or marker j,

$\mu$ is the arithmetic mean of expression values of the plurality of control samples for marker i or marker j, and $\delta$ is the standard deviation of expression values of the plurality of control samples for marker i or marker j.

According to an aspect of the invention, if $z_{a,i}$ is greater than a predetermined value v, then $z_{a,i}$ is set at

$$Z_{a,i} = V$$

and/or if $z_{a,j}$ is lower than a predetermined value -v, then

$$Z_{a,j} = -V.$$

This ensures that if individual summands $z_{a,i}$ are to substantial, they are cut at a predetermined value to give a more balanced combined score $Z_{(a)}$.

The predetermined value v can be selected according to the distribution of expression values, which usually distribute in a given statistical distribution. For example v can be selected as a certain percentile or quantile of said distribution.

According to an aspect of the invention, the method further comprises the step of classifying the sample of said patient from the outcome of the comparison in step b) into one of at least two classes.

According to an aspect of the invention, the method further comprises the step of diagnosing a disease, predicting the risk of developing a disease, or predicting an outcome of a disease in a patient suffering from or at risk of developing said disease.

According to an aspect of the invention a given marker of said plurality of markers is selected from the group consisting of DNA, RNA, mRNA, miRNA, peptide, polypeptide, protein.

According to an aspect of the invention the marker is a miRNA and the sample is selected from the group consisting of blood sample, serum sample and plasma sample.

According to an aspect of the invention the marker is a nucleic acid and the determination of the expression level in step (a) is obtained by use of a method selected from the group consisting of a Sequencing-based method, an array based method and a PCR based method.

According to an aspect of the invention the expression levels of at least 2, 3, 4, 5, 10, 20, 50 or 100 markers are determined.

The invention further relates to a computer program product useful for performing the method according to any the invention as described above, comprising

- means for receiving data representing a plurality of expression levels of a plurality of markers determined in a sample
- means for receiving data representing a reference pattern of expression levels of a plurality of control samples,
- means for mathematically combining the plurality of expression level values and comparing the plurality of expression level values to a reference pattern of expression levels of a plurality of control samples to obtain a combined score, and
- means for outputting said combined score or a classification based on said combined score.

In the attached Figures,

Figure 1 shows the formulae used in the method of the invention.

Figure 2 shows a user interface of a computer program product of the invention.

Materials and Methods

The method of the invention was used to analyze different miRNA marker combinations for differentiating between samples of Alzheimer Disease patients and healthy controls

Patient cohorts

The expression of miRNAs in peripheral blood of patients and healthy controls was determined, either by NGS or by qRT-PCR or both. Blood was obtained from patients with Alzheimer's Disease (AD) (n=106) and from healthy controls (n=22).

First, samples from AD patients (n=48) and healthy controls (n=22) were analyzed by Next-generation sequencing. For validation purposes the expression of single miRNAs was analyzed using qRT-PCR in the same samples as used for NGS, if enough RNA was available.

RNA isolation

Total RNA including miRNA was isolated using the PAXgene Blood miRNA Kit (Qiagen) following the manufacturer's recommendations. Isolated RNA was stored at -80°C. RNA integrity was analyzed using Bioanalyzer 2100 (Agilent) and concentration and purity were measured using NanoDrop 2000 (Thermo Scientific).

Library preparation and Next-Generation Sequencing

For the library preparation, 200 ng of total RNA was used per sample, as determined with a RNA 6000 Nano Chip on the Bioanalyzer 2100 (Agilent). Preparation was performed following the protocol of the TruSeq Small RNA Sample Prep Kit (Illumina). Concentration of the ready prepped libraries was measured on the Bioanalyzer using the DNA 1000 Chip. Libraries were then pooled in batches of six samples in equal amounts and clustered with a concentration of 9 pmol in one lane each of a single read flowcell using the cBot (Illumina). Sequencing of 50 cycles was performed on a HiSeq 2000 (Illumina). Demultiplexing of the raw sequencing data and generation of the fastq files was done using CASAVA v.1.8.2.

NGS data analysis

The raw illumina reads were first preprocessed by cutting the 3' adapter sequence using the programm fastx_clipper from the FASTX-Toolkit (http://hannonlab.cshl.edu/fastx_toolkit/). Reads shorter than 18 nts after clipping were removed. The remaining reads are reduced to unique reads and their frequency per sample to make the mapping steps more time efficient. For the remaining steps, we used the miRDeep2 pipeline. These steps consist of mapping the reads against the genome (hg19), mapping the reads against miRNA precursor sequences from mirbase release v18, summarizing the counts for the samples, and the prediction of novel miRNAs. Since the miRDeep2 pipeline predicts novel miRNAs per sample, the miRNAs were merged afterwards as follows: first, the novel miRNAs per sample that have a signal-to-noise ratio of more than 10 were extracted. Subsequently, only those novel miRNAs that are located on the same chromosome were merged, and both their mature forms share an overlap of at least 11 nucleotides.

Quantitative Real Time-PCR (qRT-PCR)

Out of the NGS results 7 miRNAs were selected that were deregulated in Alzheimer's Disease in comparison to healthy individuals. Five of the seven miRNAs, namely hsa-miR-5010-3p, hsa-miR-103a-3p, hsa-miR-107, hsa-let-7d-3p, and hsa-miR-532-5p were already known mature miRNAs included in miRBase, two miRNAs, namely brain-mir-112 and brain-mir-161, were newly identified and not yet included in miRBase. As endogenous control the small nuclear RNA RNU48 as used.

The miScript PCR System (Qiagen) was used for reverse transcription and qRT-PCR. A total of 200 ng RNA was converted into cDNA using the miScript Reverse Transcription Kit according to the manufacturers' protocol. For each RNA we additionally prepared $5\mu l$ reactions containing 200ng RNA and $4\mu l$ of the 5x miScript RT Buffer but no miScript Reverse Transcriptase Mix, as negative control for the reverse transcription (RT- control). The qRT-PCR was performed with the miScript SYBR® Green PCR Kit in a total volume of $20\mu l$ per reaction containing $1\mu l$ cDNA according to the manufacturers' protocol. For each miScript Primer Assay we additionally prepared a PCR negative-control with water instead of cDNA (non-template control, NTC).

Bioinformatics analysis

First the read counts were normalized using standard quantile normalization. All miRNAs with less than 50 read counts were excluded from further considerations. Next, we calculated for each miRNA the area under the receiver operator characteristic curve (AUC), the fold-change, and the significance value (p-value) using t-tests. All significance values were adjusted for multiple testing using the Benjamini Hochberg approach. The bioinformatics analyses have been carried out using the freely available tool. Furthermore, we carried out a miRNA enrichment analysis using the TAM tool (http://202.38.126.151/hmdd/tools/tam.html).

Results

Screening using high-throughput sequencing

To detect potential Alzheimer biomarkers a high-throughput sequencing of n = 22 controls samples (C), n = 48 Alzheimer patient (AD) samples was carried out. Precisely, Illumina HiSeq 2000 sequencing and multiplexed 8 samples on each sequencing lane was carried out. Thereby, 1150 of all human mature miRNAs in at least a single sample could be detected.

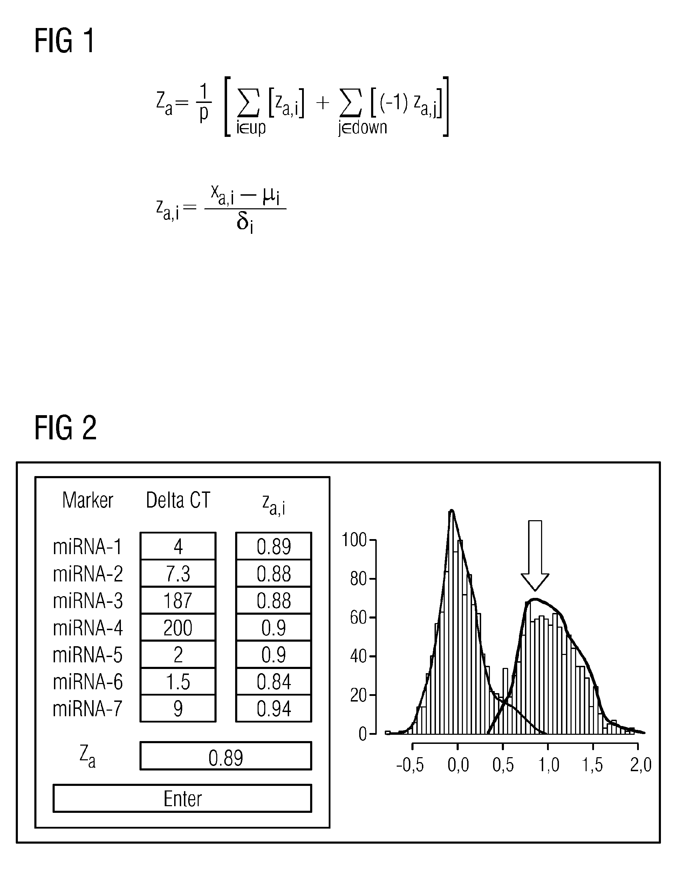

Apart from single markers, combinations of multiple markers have demonstrated a potential to improve the diagnostic accuracy. To test this hypothesis, a standard machine learning approach was applied. In a cross-validation loop, the markers with lowest significance values were stepwise added and repeatedly radial basis function support vector machines were carried out. The accuracy, specificity and sensitivity depend on the number of biomarkers. It could be shown that accuracy, specificity and sensitivity increase up to a signature number of 250 miRNAs and then converge to 90%. However, this set of miRNAs contains a significant amount of redundant biomarkers, i.e., markers that have almost identical information content to each other and are highly correlated such that even significantly smaller sets of markers can be expected to perform highly accurate distinction in Alzheimer samples and controls. We selected a signature of just 7 markers, namely the up-regulated miRNAs brain-mir-112, brain-mir-161, hsa-let-7d-3p and hsa-miR-5010-3p as well as the down-regulated markers hsa-miR-103a-3p, hsa-miR-107 and hsa-miR-532-5p. To combine the values of the 7 miRNAs in one score we calculated the average z-score using the following formula, also shown in Fig. 1:

$$Z_a = \frac{1}{p}\left[ \sum_{i \in up}[z_{a,i}] + \sum_{j \in down}[(-1)z_{a,j}] \right]$$

To compute the score for a patient a, the above formula is computed, where $z_{a,i}$ corresponds to the z-score of patient a for each up-regulated miRNAi and $z_{a,j}$ corresponds to the z-score for each patient a and each down-regulated miRNAj. The overall number of miRNAs in the signature is provided by p.

As the above formula shows z-scores can become substantially large in case of large deviations from the population mean or even more in case of small standard deviations. In this case, z-scores for single miRNAs (e.g. in the range of 5-10) would dominate the average score. Thus, it is sensible to cut the tails of the z-distribution at a sensible level. This can be achieved e.g. by cutting the single z-scores at the 5% quantiles, i.e., all z-scores were in the interval between [-1.645 and 1.645], in order to make the estimation more robust against outliers.

More precisely, $z_{a,i}$ computes as

$$z_{a,i} = \frac{x_{a,i} - \mu_i}{\delta_i}$$

where the arithmetic mean of the gene expression values for the marker i of all control cases $\mu_i$ is subtracted from the actual expression value of x patient a and divided by the standard deviation of all control cases $\delta_i$.

Fig. 2 shows an exemplary user interface of a computer program product according to the invention with an input mask to receive Delta CT values (as obtained by qRT-PCR) for miRNA markers 1 - 7. The Z-scores for each marker is calculated and the overall score is calculated as well and outputted on screen. Physicians enter the measured miRNA values and get a single score as output together with the distribution and where the patient is located in the distribution. The right distribution (under the arrow) belongs in this case to diseased patients, the other distribution to healthy controls. The actually investigated patient with an overall score of 0.89 (marked by the arrow) is classified as patient.

Example 1

In the following example, the Z score for a two marker combination is determined. The average expression

value $\mu$ and corresponding standard deviation $\delta$ is determined from 10 controls. The expression value x as determined for one individual patient sample (a) is 5 for marker 1 and 17 for marker 2 (cf. Table 2)

| | Control # | | | | | | | | | | $\mu$ | $\delta$ | X | Z |
|---|---|---|---|---|---|---|---|---|---|---|---|---|---|---|
| | 1 | 2 | 3 | 4 | 5 | 6 | 7 | 8 | 9 | 10 | | | | |
| Marker 1 | 1 | 2 | 3 | 1 | 2 | 4 | 1 | 2 | 3 | 4 | 2.3 | 1.16 | 5 | 2.329 |
| Marker 2 | 1 0 | 13 | 10 | 12 | 15 | 17 | 9 | 8 | 6 | 17 | 11.7 | 3.78 | 17 | 1.405 |

For Marker 1, $z_{a,1}$ equals: $z_{a,1} = 2,329$.
For Marker 2, $z_{a,2}$ equals: $z_{a,2} = 1,405$.
To curtail outliers, the value for $z_{a,1}$ is cut at the 5% quantiles, i.e., all z-scores were in the interval between [-1.645 and 1.645], such that $z_{a,1} = 1,645$.
The combined score $Z_a$ for the two marker signature for patient sample (a) is thus: $Z_a = \frac{1}{2}(1.625+1.405) = 1.515$.

Example 2

To compare expression value patterns of Alzheimer Disease pa(AD) patients vs. healthy controls, we selected a signature of 7 markers miRNA markers, four up-regulated miRNAs as well as the three down-regulated miRNA markers. To combine the values of the 7 miRNAs in one score we calculated the average z-score as detailed above. While we reached averaged values of 0.087 and standard deviation of 0.72 for the controls and average values of 0.22 and standard deviation of 0.74 for the MCI patients, AD patients reached a much higher score of 0.63 at a standard deviation of 0.64. Thus, the Alzheimer patients have significantly higher scores as controls, indicated by the two-tailed t-test p-value of 0.025.

Example 3

A further signature of 12 markers with limited cross-correlation was selected, including the most strongly dys-regulated markers that are less frequently dys-regulated in other diseases and show a potential to separate AD from controls. More precisely, this selected signature contained eight up-regulated miRNA markers as well as four down-regulated markers. To combine the values of the 12 miRNAs in one score the combined score was computed as discussed above. While averaged values of 0 and standard deviation of 0.39 for the controls were reached, AD patients reached a much higher score of 0.93 at a standard deviation of 0.54. Thus, the Alzheimer patients have significantly higher scores as controls, indicated by the two-tailed t-test p-value of $3.7*10^{-11}$ in case of AD versus controls.

Example 1

In the following example, five further signatures were tested for their ability to separate AD from controls (see Table 2). Each of the five signature sig#1 to sig#5 is composed of five individual miRNA markers. Each signature was calculated for a first cohort ("Z-Score AD") and for a second replication cohort ("Z-Score AD 2nd cohort"). The Z-Score values are the mean Z-score for the entire cohort. Due to "flooring", i.e. cutting off values for za,i above 1.645 and below -1.645 the values of the controls does not equal 0, but deviates between -0.012 and -0.028.

Table 2 Combined scores (Z-Scores) for five signatures separating AD from controls.

| signature | Z-Score AD | Control | Z-Score AD 2nd cohort | miRNA 1 | miRNA 2 | miRNA 3 | miRNA 4 | miRNA 5 |
|-----------|-----------|---------|----------------------|---------|---------|---------|---------|---------|
| sig #1 | 1.123 | -0.019 | 1.19 | hsa-miR-1285-5p | brain-mir-112 | hsa-miR-5010-3p | hsa-miR-151a-3p | hsa-let-7f-5p |
| sig #2 | 1.054 | -0.012 | 1.281 | hsa-miR-3127-3p | hsa-miR-1285-5p | hsa-miR-425-5p | hsa-miR-148b-5p | hsa-miR-144-5p |
| sig #3 | 1.101 | -0.028 | 1.137 | hsa-miR-3127-3p | hsa-miR-3157-3p | hsa-miR-148b-5p | hsa-miR 151a-3p | hsa-miR-144-5p |
| sig #4 | 1.097 | -0.015 | 1.325 | hsa-miR-3127-3p | hsa-miR-1285-5p | hsa-miR-425-5p | hsa-miR-151a-3p | hsa-miR-144-5p |
| sig #5 | 1.111 | -0.02 | 1.187 | hsa-miR-1285-5p | brain-mir-112 | hsa-miR-5010-3p | hsa-miR-151a-3p | hsa-let-7a-5p |

**Claims**

1. A method of evaluating a sample of a patient, said method comprising the steps of:

   a) determining in said sample an expression level of a plurality of markers to obtain a plurality of expression level values,
   b) mathematically combining the plurality of expression level values and comparing the plurality of expression level values to a reference pattern of expression levels of a plurality of control samples,
   wherein said step of mathematically combining and comparing is performed by calculating a combined score using the formula

$$Z_{(a)} = 1/p \left[ \sum [z_{ai}] \ (i \in \text{upregulated}) + \sum (-1) [z_{aj}] \ (j \in \text{down-regulated}) \right]$$

   where $Z_{(a)}$ is the combined score,
   $z_{a,i}$ is the score of the patient a for each up-regulated marker i,
   $z_{a,j}$ is the z-score for each patient a and each down-regulated marker j,
   p is the overall number of markers of said plurality of markers,
   and wherein

$$z_{a,i} = (x_{ai} - \mu_i)/\delta_i$$

   and

$$z_{a,j} = (x_{aj} - \mu_j)/\delta_j$$

   where x is the actual expression value of patient a for marker i or marker j,
   $\mu$ is the arithmetic mean of expression values of the plurality of control samples for marker i or marker j, and
   $\delta$ is the standard deviation of expression values of the plurality of control samples for marker i or marker j.

2. The method of claim 1, wherein, if za,i is greater than a predetermined value *v*, then *za,i* is set at

$$z_{a,i} = V$$

   and/or if za,j is lower than a predetermined value -*v*, then

$$z_{a,j} = -V.$$

3. The method according to any of the preceding claims, further comprising the step of classifying the sample of said patient from the outcome of the comparison in step b) into one of at least two classes.

4. The method according to any of the preceding claims, further comprising the step of diagnosing a disease, predicting the risk of developing a disease, or predicting an outcome of a disease in a patient suffering from or at risk of developing said disease.

5. The method according to any of the preceding claims, wherein a given marker of said plurality of markers is selected from the group consisting of DNA, RNA, mRNA, miRNA, peptide, polypeptide, protein.

6. The method according to claim 4, wherein the marker is a miRNA and the sample is selected from the group consisting of blood sample, serum sample and plasma sample.

7. The method according to any of the preceding claims, wherein the marker is a nucleic acid and the determination of the expression level in step (a) is obtained by use of a method selected from the group consisting of a Sequencing-based method, an array based method and a PCR based method.

8. The method according to any of the preceding claims, wherein the expression levels of at least 2, 3, 4, 5, 10, 20, 50 or 100 markers are determined.

9. A computer program product useful for performing the method according to any of claims 1 to 8, comprising

- means for receiving data representing a plurality of expression levels of a plurality of markers determined in a sample
- means for receiving data representing a reference pattern of expression levels of a plurality of control samples,
- means for mathematically combining the plurality of expression level values and comparing the plurality of expression level values to a reference pattern of expression levels of a plurality of control samples to obtain a combined score, and
- means for outputting said combined score or a classification based on said combined score.

# FIG 1

$$Z_a = \frac{1}{p} \left[ \sum_{i \in up} \left[ z_{a,i} \right] + \sum_{j \in down} \left[ (-1)\, z_{a,j} \right] \right]$$

$$z_{a,i} = \frac{x_{a,i} - \mu_i}{\delta_i}$$

# FIG 2

| Marker | Delta CT | $z_{a,i}$ |
|---|---|---|
| miRNA-1 | 4 | 0.89 |
| miRNA-2 | 7.3 | 0.88 |
| miRNA-3 | 187 | 0.88 |
| miRNA-4 | 200 | 0.9 |
| miRNA-5 | 2 | 0.9 |
| miRNA-6 | 1.5 | 0.84 |
| miRNA-7 | 9 | 0.94 |
| $Z_a$ | 0.89 | |
| Enter | | |

Europäisches
Patentamt

European
Patent Office

Office européen
des brevets

# EUROPEAN SEARCH REPORT

Application Number

EP 12 19 2985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 328 105 A2 (SYSMEX CORP [JP]) 1 June 2011 (2011-06-01) * p.4, paragraph 13, 15 p. 5, paragraph 19 p.6, paragraph 29-30; claims 1,2 * | 1,3-9 | INV. G06F19/20 G06F19/24 |
| A | WO 2010/056993 A2 (UNIV EMORY [US]; MORENO CARLOS [US]; OSUNKOYA ADEBOYE [US]; ZHOU WEI) 20 May 2010 (2010-05-20) * the whole document * | 1-9 | |
| A | US 2010/184063 A1 (TSAO MING-SOUND [CA] ET AL) 22 July 2010 (2010-07-22) * the whole document * | 1-9 | |
| A | WO 2012/075069 A2 (DANA FARBER CANCER INST INC [US]; HAHN WILLIAM C [US]; TAMAYO PABLO [U) 7 June 2012 (2012-06-07) * the whole document * | 1-9 | |
| A,D | KELLER ANDREAS ET AL: "Toward the blood-borne miRNome of human diseases.", NATURE METHODS, vol. 8, no. 10, October 2011 (2011-10), pages 841-843, XP002694322, ISSN: 1548-7105, DOI: 10.1038/NMETH.1682 * the whole document * | 1-9 | TECHNICAL FIELDS SEARCHED (IPC) G06F G01N |

The present search report has been drawn up for all claims

| Place of search | Date of completion of the search | Examiner |
|---|---|---|
| Munich | 3 April 2013 | Schmitt, Constanze |

EPO FORM 1503 03.82 (P04C01)

## ANNEX TO THE EUROPEAN SEARCH REPORT
## ON EUROPEAN PATENT APPLICATION NO.

EP 12 19 2985

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2013

| Patent document cited in search report | | Publication date | Patent family member(s) | | Publication date |
|---|---|---|---|---|---|
| EP 2328105 | A2 | 01-06-2011 | CN 102051412 A | | 11-05-2011 |
| | | | EP 2328105 A2 | | 01-06-2011 |
| | | | JP 2011092137 A | | 12-05-2011 |
| | | | US 2011106739 A1 | | 05-05-2011 |
| WO 2010056993 | A2 | 20-05-2010 | US 2011230361 A1 | | 22-09-2011 |
| | | | WO 2010056993 A2 | | 20-05-2010 |
| US 2010184063 | A1 | 22-07-2010 | NONE | | |
| WO 2012075069 | A2 | 07-06-2012 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. KELLER et al.** *Nat Methods,* 2011, vol. 8 (10), 841-3